# EUROPÄISCHE PATENTSCHRIFT

(11) **EP 0 923 956 B1**
(45) Veröffentlichungstag und Bekanntmachung des Hinweises auf die Patenterteilung: **23.01.2002**
(21) Anmeldenummer: 98119895.5
(22) Anmeldetag: 21.10.1998
(51) Int. Cl.: A61M 5/158

(54) **Infusionskatheter**
Infusion catheter
Cathéter de perfusion

(30) Priorität: 19.12.1997 DE 29722447 U
(43) Veröffentlichungstag der Anmeldung: 23.06.1999
(73) Patentinhaber: Clinico GmbH & Co. Verwaltungs- und Beteiligungs-KG, D-36251 Bad Hersfeld (DE)
(72) Erfinder: Heinzerling, Jörg Dipl.-Wirtschaftsingenieur, 36251 Bad Hersfeld (DE)
(74) Vertreter: von Raffay, Vincenz, Dipl.-Ing.

(56) Entgegenhaltungen:
- EP-A- 0 168 289
- DE-U- 29 503 099
- US-A- 4 632 671
- US-A- 4 645 495
- US-A- 5 545 143
- US-A- 5 693 018

## Beschreibung

Die Erfindung betrifft einen Infusionskatheter nach dem Oberbegriff des Anspruches 1.

Derartige bekannte Infusionskatheter dienen der Medikamentenverabreichung. Sie verbleiben über einen längeren Zeitraum am Patienten. Um die Traumatisierung des Patienten beim Einstechen zu reduzieren und das Medikamentenrestvolumen zu minimieren, sowie eine schnelle Wundheilung zu ermöglichen, werden die Querschnitte der eingesetzten Kanülen immer geringer. Hierdurch besteht die wachsende Gefahr des Verbiegens oder Abknickens der Kanüle. Ein derartiger Infusionskatheter ist aus der DE29503099 U bekannt.

Aus der US 5,963,018 A ist ein Infusionskatheter nach dem Oberbegriff des Anspruches 1 bekannt. Dieser Infusionskatheter weist eine spiralförmig geformte Kanüle auf, wobei das Einstechende senkrecht aus der durch die Spirale gebildeten Ebene aus der auf dem Patienten aufliegenden Auflagefläche austritt. Die Spirale und der Abschnitt des Einstechendes innerhalb des Kunststoffkörpers sind von einem Kanal umgeben, dessen Querschnitt im Verhältnis zu dem Querschnitt der Kanüle groß ist. Die Kanüle kann sich in sämtlichen möglichen drei Dimensionen frei bewegen. Befestigt ist die Kanüle in dem Kunststoffkörper dadurch, daß ein Teil der Spirale, nämlich derjenige, der von dem Einstechende entfernt liegt, in eine Gießmasse eingebettet ist, so daß dieser Teil der Spirale starr in dem entsprechenden Abschnitt des Kanales befestigt ist. Der freie Abschnitt der Spirale und das Einstechende sind wie gesagt in sämtlichen drei möglichen Richtungen bewegbar. Durch diese Beweglichkeit des Einstechendes ist es möglich, daß sich das Einstechende relativ zu der Auflagefläche bewegt, wenn Kräfte auf dieses Einstechende durch Bewegung des Patienten ausgeübt werden.

Der Erfindung liegt nun die Aufgabe zugrunde, einen Infusionskatheter der eingangs genannten Art zu schaffen, der zwar eine Kanüle mit einem geringen Querschnitt aufweist, bei dem aber die Gefahr des Verbiegens oder Brechens der Kanüle, insbesondere beim Einstechen, gering gehalten wird.

Diese Aufgabe wird grundsätzlich durch das Kennzeichen des Anspruches 1 gelöst.

Erfindungsgemäß tritt das Einstechende nicht eingespannt ohne Übergang aus dem Kunststoffkörper aus, sondern durch die Ausbildung der Entlastungsnut wird dafür gesorgt, daß die auf das Einstechende übertragenen Kräfte als Biegekräfte und Torsionskräfte allmählich in den Kunststoffkörper übergeleitet werden. Entsprechendes gilt für die Übertragung der Kräfte auch beim Tragen durch den Patienten. Biegekräfte quer zur Längsachse des Einstechendes werden in dem abgewinkelten Abschnitt der Kanüle in Torsionskräfte umgewandelt. Auch dieses geschieht nicht plötzlich, sondern mit allmählichem Übergang.

Insbesondere ist der Infusionskatheter so ausgebildet, wie in den Ansprüchen 2 bis 4 angegeben. Da die Entlastungsnut, d.h. bei der bevorzugten Ausführungsform der Ringraum, bis in den Bereich der Kanülenabwinklung hineinreicht, ergibt sich für die Kanüle ein Drehpunkt der die allmähliche Übertragung und Umwandlung der Biegekräfte unterstützt.

Wenn der Infusionskatheter so ausgebildet ist, wie in Anspruch 5 angegeben, dann wird einer Verkeimung entgegengewirkt.

Im folgenden wird die Erfindung unter Hinweis auf die Zeichnung an Hand verschiedener Ausführungsbeispiele näher erläutert.

Es zeigt:
- Fig. 1: einen Längsschnitt durch eine Ausführungsform eines Infusionskatheters nach der Erfindung;
- Fig. 2: einen Querschnitt durch die Ausführungsform der Fig. 1;
- Fig. 3: einen der Fig. 1 entsprechenden Längsschnitt durch eine abgewandelte Ausführungsform; und
- Fig. 4: einen den Fig. 1 und 3 entsprechenden Längsschnitt durch eine weitere Ausführungsform.

Die in der Zeichnung dargestellten Infusionskatheter bestehen aus einem thermoplastischen Kunststoffkörper 2, in dem eine Kanüle 1 eingebettet und formschlüssig mit diesem verbunden ist. Das eine Ende der Kanüle 1, das aus einem Ansatz 7 austritt, ist mit einem Zuleitungsschlauch 3 dicht verbunden.

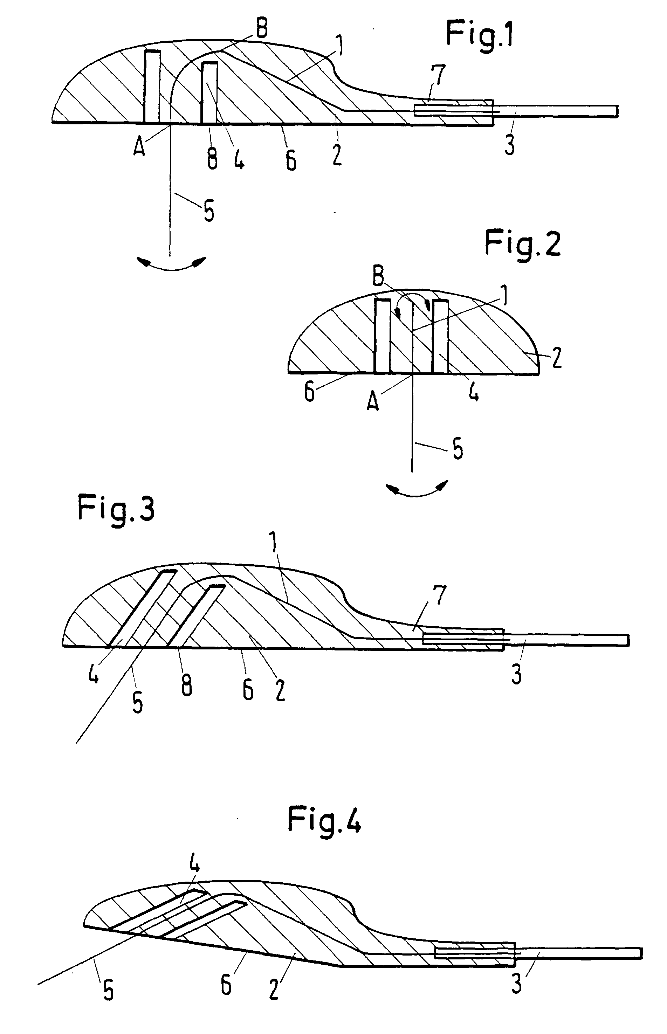

Das andere Ende der Kanüle 1, das das Einstechende 5 bildet, tritt aus der Auflagefläche 6 des Kunststoffkörpers 2 aus, und zwar bei der Ausführungsform nach den Fig. 1 und 2 im wesentlichen rechtwinklig. In dem Kunststoffkörper 2 ist die Kanüle 1 abgewinkelt, wobei der entstehende Winkel ungefähr ein rechter Winkel ist. Diese Stelle ist mit B bezeichnet. Die Austrittsstelle des Einstechendes 5 der Kanüle 1 ist mit A bezeichnet.

Ausgehend von der Auflagefläche 6 ist um die Kanüle 1 herum eine Entlastungsnut in Form eines Ringraumes 4 vorgesehen. Dieser Ringraum 4 verläuft bis in die Nähe der gegenüberliegenden Fläche des Kunststoffkörpers 2. Lediglich dort, wo die abgewinkelte Kanüle 1 hindurchtritt, ist der Ringraum 4 etwas kürzer.

Die Ausführungsform nach den Fig. 3 und 4 unterscheidet sich dadurch von derjenigen nach den Fig. 1 und 2, daß das Einstechende 5 nicht genau in einem rechten Winkel aus der Auflagefläche 6 austritt, sondern einen Winkel von etwas mehr als 90° zu dem Ende des Kunststoffkörpers 2 aufweist, an dem der Ansatz 7 vorgesehen ist. Bei der Ausführungsform nach Fig. 4 ist der Ansatz 7 nicht genau parallel zu der Auflagefläche 6. Die Auflagefläche ist bei dieser Ausführungsform etwas geneigt zu dem Ansatz 7 und damit zu dem dort austretenden Ende der Kanüle, das zur Verbindung mit der Zuleitung 3 vorgesehen ist.

Mit 8 ist eine dünne Schutzhaut bezeichnet, die den Ringraum im Bereich der Auflagefläche 6 abdeckt, um hier eine Verkeimung zu verhindern.

Beim Betrachten der Zeichnung ist ohne weiteres erkennbar, daß Biegekräfte, die von dem Einstechende 5 auf den Punkt A übertragen werden, allmählich bis zum Punkt B übergehen. Entsprechendes gilt für Torsionskräfte, die beim Einstechen und Tragen auftreten. Biegekräfte, die auf das Einstechende 5 ausgeübt werden, werden im Punkt B teilweise in Torsionskräfte allmählich umgeleitet.

## Patentansprüche

1. Infusionskatheter mit einem Kunststoffkörper (2) mit Kanüle (1), deren eines Ende mit einer Zuleitung (3) verbunden ist und deren anderes aus der auf dem Patienten aufliegenden Auflagefläche (6) austretendes Ende das Einstechende (5) bildet, **dadurch gekennzeichnet, daß** die Kanüle (1) in dem Kunststoffkörper (2) eingebettet und formschlüssig von diesem umgeben ist und
daß der Kunststoffkörper ausgehend von der Auflagefläche (6) um die Kanüle herum eine Entlastungsnut in Form eines Ringraumes (4) aufweist.

2. Infusionskatheter nach Anspruch 1, **dadurch gekennzeichnet, daß** die Achse des Ringraumes (4) mit der Achse der Kanüle (1) zusammenfällt.

3. Infusionskatheter nach Anspruch 1 oder 2, **gekennzeichnet durch** einen im wesentlichen parallel zu der Auflagefläche (6) verlaufenden Ansatz (7), aus dem das mit der Zuleitung (3) verbundene Ende der ungefähr um 90° abgewinkelten Kanüle (1) zur Verbindung mit der Zuleitung austritt.

4. Infusionskatheter nach Anspruch 3, **dadurch gekennzeichnet, daß** der Ringraum (4) von der Auflagefläche (6) im wesentlichen bis zur gegenüberliegenden Fläche des Kunststoffkörpers (2) hindurch verläuft und lediglich am Durchtritt (bei B) der abgewinkelten Kanüle (1) etwas kürzer ist.

5. Infusionskatheter nach einem oder mehreren der vorstehenden Ansprüche, **dadurch gekennzeichnet, daß** der Ringraum an der Auflagefläche (6) durch eine dünne Schutzhaut (8) verschlossen ist.

## Claims

1. Infusion catheter with a plastic body (2) having a cannula (1), whose one end is connected to a feed line (3) and whose other end passing out of the bearing surface (6) resting on the patient forms the insertion end (5), **characterized in that** the cannula (1) is embedded in the plastic body and is positively surrounded by the latter and that, starting from the bearing surface (6), around the cannula the plastic body has a relief groove in the form of an annular space (4).

2. Infusion catheter according to claim 1, **characterized in that** the axis of the annular space (4) coincides with the axis of the cannula (1).

3. Infusion catheter according to claim 1 or 2, **characterized by** a shoulder (7) running substantially parallel to the bearing surface (6) from which passes the end of the cannula (1) angled by approximately 90° for connection with the feed line (3).

4. Infusion catheter according to claim 3, **characterized in that** the annular space (4) passes from the bearing surface (6) substantially to the opposite surface of the plastic body (2) and is only somewhat shorter at the passage (at B) of the angled cannula (1).

5. Infusion catheter according to one or more of the preceding claims, **characterized in that** the annular space is closed at the bearing surface (6) by a thin protective skin (8).

## Revendications

1. Cathéter à infusion avec un corps en matière synthétique (2) équipé d'une canule (1), dont une extrémité est reliée à une conduite d'amenée (3) et dont l'autre extrémité, sortant de la surface de pose (6), reposant sur le patient, forme l'extrémité à enficher (5), **caractérisé en ce que** la canule (1) est noyée dans le corps en matière synthétique (2) et entourée par celui-ci avec une liaison à ajustement de forme et **en ce que** le corps en matière synthétique, en partant de la surface de pose (6) autour de la canule, présente une rainure de décharge ayant la forme d'un espace annulaire (4).

2. Cathéter à infusion selon la revendication 1, **caractérisé en ce que** l'axe de l'espace annulaire (4) coïncide avec l'axe de la canule (1).

3. Cathéter à infusion selon la revendication 1 ou 2, **caractérisé par** un appendice (7) s'étendant sensiblement parallèlement à la surface de pose (6), appendice d'où sort l'extrémité, reliée à la conduite d'amenée (3), de la canule (1) coudée à peu près de 90°, pour assurer la liaison à la conduite d'amenée.

4. Cathéter à infusion selon la revendication 3, **caractérisé en ce que** l'espace annulaire (4) s'étend depuis la surface de pose (6) sensiblement jusqu'à la surface opposée du corps en matière synthétique (2) et est un peu plus court uniquement au passage (en B) de la canule (1) coudée.

5. Cathéter à infusion selon l'une ou plusieurs des revendications précédentes, **caractérisé en ce que** l'espace annulaire ménagé dans la surface de pose (6) est fermé par une peau protectrice (8) mince.
